Europäisches Patentamt

European Patent Office

Office européen des brevets

(11) Publication number: **0 468 123 A1**

# EUROPEAN PATENT APPLICATION

(21) Application number: **90500071.7**

(22) Date of filing: **23.07.90**

(51) Int. Cl.5: **A61F 11/08**

(43) Date of publication of application:
**29.01.92 Bulletin 92/05**

(84) Designated Contracting States:
**BE DE FR GB GR IT LU NL**

(71) Applicant: **JOVAN, S.L.**
**C/ Cirera, 25, 1**
**E-08240 Manresa (Barcelona)(ES)**

(72) Inventor: **Donati Bagnoli, Claudio**
**c/ Tres Roures, No. 12, 3o 4a**
**E-08240 Manresa (Barcelona)(ES)**

(74) Representative: **Aguilar Forment, Domenec (ES)**
**c/o Aguilar & Revenga Puerto Principe, 7**
**E-08027 Barcelona(ES)**

(54) Improvements in the production of ear plugs.

(57) The process for the production of ear plugs consists of forming a plastic mass with a base of synthetic resins and plasticising agents, hot-mixed in a water bath, in an open reactor, by means of regulated stirring. Plasticising agents, emulsifiers, officinal triethanolamine, bactericides and a freeze-dried heterologous collagen are added to this mass.

The plastic mass retains its mouldability between 4 and 70oC, and it is waterproof and soundproof. It is fitted at the entrance of the auditory canal to provide protection against noise and water.

EP 0 468 123 A1

The purpose of this invention, as expressed in the introduction to this descriptive report, consists of some improvements in the production of ear plugs, to be used particularly for bolcking the ear by means of a small plug placed in the external auditory canal that intercepts at least two of the possible harmful factores for the organ in question, namely water and noise.

The use of ear plugs is mainly recommended in very noisy environments and for bathing at the beach, swimming pools, etc., as a prevention against the harmful results of noise and against possible disorders caused by waters that have been polluted, either by chemical or industrial waste, accidents in rivers or at sea, epidemics, etc., which may cause real and very often irreversible problems to these polluted water.

Moreover, an effective ear plug, apart from blocking or preventing harmful external factors, such as those mentioned above, should obviously not cause tje user any other kind of harm or disconform, because in this case its use would not be justified.

All ear plugs currently on the market in this sector have in one way or another, some disadvantages, such as poor functional perfomance, or else they cause harmful and distressful side effects.

Specifically, it is difficult for latex foam rubber plugs to be moulded to the auditory canal, and their elastic non reducible structure tends to press on the walls that hold them, causing real pain.

Polyurethane foam rubber plugs, more pliant than the former, are also useless, as they absorb water, and when they are removed, they allow the water to penetrate inside the ear, thus cancelling out their insulating effect.

Plugs made of plasticised synthetic rubber or silicone are brittle when moulded, particularly if they are wet, and so they should be discarded. Its lack of sensitivity to body heat is another disadvantage of this type of plug, since it does not allow environmental adaptability in the auditory canal, thus causing pain.

The traditional well-know cotton wool and paraffin plugs are not heat-resistant, even to body heat, and they exude their deliquescent and oily substances, thus rather unpleassantly soiling the ear or the hands that touch them.

When these plugs are exposed to more extreme temperatures, as for example when exposed to the sun at the beach, swimming pools, etc., they soften to such an extent that they become unusable.

In view of these disadvantages, the ideal solution is clearly to provide an ear plg that:

- gives effective insulation from acoustic stress brought on by noise, without being excessive and thus causing claustrophobic fear.
- moulds easily to the walls of the auditory canal by careful softening as a result of body temperature and by gentle moulding with the hands.
- does not harden when in contact with water or cold, so as not to --cause the pain that results from inserting something hard into an - extremely sensitive and delicate area.
- that contains no wax, fat or oil that are sensitive to the extreme-temperatures that may be encountered at the beach, swimming pools, etc.
- that softens gently with body heat or by prolonged exposure to the-sun, or when immersed in hot water, without disagreeable oily exudations.
- that, by containing disifenctant substances, effectively prevents - disorders from water pollution, and if possible enables these substances to retain their antiseptic effect, even after repeated use.
- that can be washed easily, enabling it to be used repeatedly, thus-helping the household economy.

In view of the above, a description is given of the production of an ear plug that has the above-mentioned beneficial qualities, thus providing guaranteed efficiency and total elimination of the harmful side effects.

To this end, the ear plug in question has a mass with a base of synthetic resins and plasticising agents, hot-mixed in a water bath, in an open reactor, by means of regulated stirring. Plasticising agent, emulsifiers, official triethanolamine, bactericides and a freeze-dried heterologous collagen are added to this mass.

The plastic mass produced retains its mouldability between 4 and 70oC, and is waterproof and soundproof.

The synthetic resins used are modified acrylic polymethacrylate resin, copolymerised vinyl acetate resin and fluorocarbon-modified and micronised polyethylene resin. The plasticising agents used are synthetic camphor, diethyl phthalate and dioctyl phthalate.

The emulsifiers used are non-ionic in type, with a high degre of oxyethylation and water-receptive; such as polyglycol monylphenol ether, and water-repellent in type, such as fatty alcohol and polyglycol ether.

The bactericides are broad-spectrum gram positive and gram negative in type, such as benzalkonium chloride and myristyltrimethylammonium bromide.

The quantitative composition, given as a purely illustrative and not restrictive example, of the practical possibilities for making the plug material is as follows:

```
Plasticising agents: – Diethyl Phthalate. . . . . . . . . .  0.460 g.
                     – Dioctyl Phthalate. . . . . . . . . .  0.460 g.
Resins:              – Polyacrylic. . . . . . . . . . . . .  0.460 g.
                     – Polyvinyl. . . . . . . . . . . . . .  0.430 g.
                     – Polyethylene . . . . . . . . . . . .  0.080 g.
Emulsifiers:         – Fatty alcohol and
                       polyglycol ether . . . . . . . . . .  0.070 g.
```

```
                     – Polyglycol monylphenol
                       ether. . . . . . . . . . . . . . . .  0.120 g.
Stearic Acid. . . . . . . . . . . . . . . . . . . . . . . .  0.295 g.
Synthetic Camphor . . . . . . . . . . . . . . . . . . . . .  0.040 g.
Sodium Stearate . . . . . . . . . . . . . . . . . . . . . .  o.195 g.
Official Triethanolamine. . . . . . . . . . . . . . . . . .  0.075 g.
Antiseptic Composition. . . . . . . . . . . . . . . . . . .  0.315 g.
```

The antiseptic composition is made up of:

```
Freeze–dried heterologous collagen. . . . . . . . . . . . .  0.265 g.
Water . . . . . . . . . . . . . . . . . . . . . . . . . . .  0.490 g.
Isopropyl Alcohol . . . . . . . . . . . . . . . . . . . . .  0.300 g.
Benzalkonium Chloride . . . . . . . . . . . . . . . . . . .  0.100 g.
Myristyltrimethylammonium Bromide . . . . . . . . . . . . .  0.100 g.
Carmine red . . . . . . . . . . . . . . . . . . . . . . . .  0.010 g.
```

## Claims

1. "IMPROVEMENTS IN THE PRODUCTION OF EAR PLUGS", of the type to be fitted at the entrance of the external auditory canal. The improvements are characterised essentially in that they consists of forming a plastic mass with a base of synthetic resins and plasticising agents, hot-mixed in a water bath, in an open reactor, by means of regulated stirring. Plasticising agents, emulsifiers, official triethanolamine, bactericides and a freeze-dried heterologous collagen are added to this mass.

2. "IMPROVEMENTS IN THE PRODUCTION OF EAR PLUGS", as per previous claim, characterised essentially in that the plastic mass retains its mouldability between 4 and 7OoC, and is waterproof and soundproof

3. "IMPROVEMENTS IN THE PRODUCTION OF EAR PLUGS", as per previous claims, characterised essentially in that the synthetic resins used are acrylic polymethacrylate-modified resin, copolymerised vinyl acetate resin and fluorocarbon-modified and micronised polyethylene resin.

4. "IMPROVEMENTS IN THE PRODUCTION OF EAR PLUGS", as per previous claims, characterised essentially in that the plasticising agents used are synthetic camphor, diethyl phthalate and dioctyl phthalate.

5. "IMPROVEMENTS IN THE PRODUCTION OF EAR PLUGS", as per previous claims, characterised essentially in that the emulsifiers used are non-ionic in type with a high degree of oxyethylation and water-receptive, such as polyglycol monylphenol ether, and water-resistant, such as fatty alcohol and polyglycol ether.

6. "IMPROVEMENTS IN THE PRODUCTION OF EAR PLUGS", as per previous claims, characterised essentially in that the bactericides used are broad-spectrum gram positive and gram negative in type, such as benzalkonium chloride and myristyltrimethylammonium bromide.

7. "IMPROVEMENTS IN THE PRODUCTION OF EAR PLUGS", as per previous claims, characterised essentially in that the freeze-dried heterologous collagen contains sodium ethylmercurothiosalicylate.

8. "IMPROVEMENTS IN THE PRODUCTION OF EAR PLUGS", as per previous claims, characterised essentially in that a dye, such as carmine, is added to the plastic mass.

9. "IMPROVEMENTS IN THE PRODUCTION OF EAR PLUGS", as per previous claims, characterised essentially in that the total weight composition is made up of 25 to 35% synthetic resins, 25 to 35% plasticising agents, the emulsifiers are 5 to 10%, while Official Triethanolamine comprises between 1 and 3%, freeze-dried heterolous collagen between 4 and 30% and the antiseptic composition is between 0.1 and 0.5%.

European
Patent Office

EUROPEAN SEARCH
REPORT

Application Number

EP 90 50 0071

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WORLD PATENTS INDEX, week 8947, 1989, classes A96,D22,P32, accession no. 89-341981 [47], Derwent Publications Ltd, London, GB; & ES-A-2 007 794 (S.L. JOVAN) 01-07-1989 * No. 89-341981 * | 1-9 | A 61 F 11/08 |
| A | DE-C-831 748  (C. GÖHLICH) * Page 2, lines 22-31,39-45; claim 2 * | 1,3,8-9 | |
| A | DE-U-1 922 132  (F.W. HILLMANN) * Abstract * | 1,9 | |
| A | EP-A-0 108 728  (AMPLISILENCE) * Page 6, line 27 - page 7, line 28 * | 1,3,9 | |
| A | GB-A-1 433 836  (P.H. KITREDGE) * Claim 3 * | 2 | |

| TECHNICAL FIELDS SEARCHED (Int. Cl.5) |
|---|
| A 61 F |

The present search report has been drawn up for all claims

| Place of search | Date of completion of search | Examiner |
|---|---|---|
| The Hague | 22 January 91 | NICE P.R. |